(19) Europäisches Patentamt / European Patent Office / Office européen des brevets

(11) **EP 3 137 164 B1**

(12) **EUROPÄISCHE PATENTSCHRIFT**

(45) Veröffentlichungstag und Bekanntmachung des Hinweises auf die Patenterteilung:
**16.05.2018  Patentblatt 2018/20**

(21) Anmeldenummer: **15725040.8**

(22) Anmeldetag: **28.05.2015**

(51) Int Cl.:
***A61N 1/375*** *(2006.01)*

(86) Internationale Anmeldenummer:
**PCT/EP2015/061908**

(87) Internationale Veröffentlichungsnummer:
**WO 2015/193077 (23.12.2015 Gazette 2015/51)**

(54) **GEHÄUSE FÜR EIN MEDIZINISCHES IMPLANTAT**

HOUSING FOR A MEDICAL IMPLANT

BOÎTIER POUR IMPLANT À USAGE MÉDICAL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.06.2014  DE 102014009136**

(43) Veröffentlichungstag der Anmeldung:
**08.03.2017  Patentblatt 2017/10**

(73) Patentinhaber: **Forschungszentrum Jülich GmbH 52425 Jülich (DE)**

(72) Erfinder: **HAUPTMANN, Christian 82319 Starnberg (DE)**

(74) Vertreter: **Manitz Finsterwald Patentanwälte PartmbB Martin-Greif-Strasse 1 80336 München (DE)**

(56) Entgegenhaltungen:
**EP-A1- 2 392 382      EP-A2- 2 123 325 US-A1- 2007 260 294**

**Beschreibung**

Die Erfindung betrifft ein Gehäuse für ein medizinisches Implantat.

Erkrankungen des Zentralnervensystems, wie beispielsweise Epilepsie, die Parkinsonsche Erkrankung oder Zwangserkrankungen, werden u.a. mittels direkter elektrischer Stimulation des Gehirns therapiert. Dazu werden Elektroden in die Zielareale implantiert und unter der Haut mit entsprechenden Implantat-Systemen elektrisch verbunden. Über diese Implantatsysteme werden elektrische Reize an das Zielareal übertragen. Bei der elektrischen Stimulation ist insbesondere die Betrachtung der Ladungsdichte und damit der Ladungsmenge pro Puls ein wichtiges Kriterium, um dauerhafte Schädigungen des Gewebes im Verlauf der therapeutischen Stimulation zu vermeiden. Geräte für die tiefe Hirnstimulation werden in "Deep Brain Stimulation Devices: A Brief Technical History and Review", Robert J. Coffey, 2008, Artificial Organs 33(3), 208-220 beschrieben. In ähnlicher Weise werden beispielsweise Herzrhythmusstörungen durch Herzschrittmacher behandelt, bei denen ebenfalls Implantate in den Körper eingesetzt werden.

Nach dem Stand der Technik wird die Übertragung der Ladungsmenge durch einen Kopplungskondensator limitiert. Pro Stimulationskontakt wird beispielsweise ein Kopplungskondensator benötigt. Die Kapazität beträgt typischerweise 100 nF und mehr, beispielsweise 1000 nF, um die Ladungsübertragung auf beispielsweise 1 $\mu$C zu limitieren.

Neuere Elektroden nach dem Stand der Technik sehen eine große Zahl von Elektrodenkontakten vor, beispielsweise 40 oder 80 Kontakte, wie sie beispielsweise von H.C.F. Martens et.al in "Spatial steering of deep brain stimulation volumes using a novel lead design", Clinical Neurophysiology, (2011), 122, 558-566 beschrieben werden.

Bisher werden üblicherweise Einzelkondensatoren oder ein Array von Kondensatoren für die Realisierung der Kopplungskondensatoren verwendet. Bei den Kondensatoren handelt es sich meist um Keramik-basierte Kondensatoren mit einer Kapazität von beispielsweise 100 nF oder mehr. Die Größe der Kapazität wird im Wesentlichen von der Versorgungsspannung des Implantats, von der Oberfläche der Stimulationskontakte und von den Anforderungen an die Effizienz des Implantats bestimmt. Wird eine höhere Versorgungsspannung oder eine kleinere Kontaktfläche gewählt, so kann die Kapazität kleiner gewählt werden.

Die Kabeldurchführung aus dem Inneren des Implantats zu den Anschlüssen der Elektrode wird häufig über die Integration eines Keramikbauteils in eine Öffnung des Gehäuses, das meist aus Titan besteht, realisiert (Feedthrough). Bei konventionellen Kabeldurchführungen werden Keramikscheiben im Titan-Gehäuse verpresst.

Die Vorrichtungen nach dem Stand der Technik haben den Nachteil, dass eine große Anzahl von Bauteilen eingesetzt werden muss, wobei ein hoher Platzbedarf besteht. Weiterhin stellt der Ort der Kabeldurchführung einen kritischer Bereich dar, der Ort einer Leckage sein kann und somit ein Sicherheitsrisiko darstellt, an dem Komplikationen oder gar Schädigungen des Patienten durch das Eindringen von Körperflüssigkeit auftreten können.

Mit den Vorrichtungen nach dem Stand der Technik wird die Sicherheit beeinträchtigt und die Implantate sind groß, was sowohl für den Patienten als auch für den Arzt Beeinträchtigungen in der Verwendung der Implantate zur Folge hat. Für den Ingenieur stellen sich Probleme in der Konstruktion, die nur schwer gelöst werden können. Die Baugröße des Implantats wird im Wesentlichen von der Batterie, den Kopplungskondensatoren, der Elektronik, dem Adapter und der Kabeldurchführung bestimmt. Ein großes Implantat ist auf Grund der Größe von außen sichtbar. Die große Anzahl von Kopplungskondensatoren nimmt einen sehr großen Raum innerhalb des Implantats ein und verhindert so die Verkleinerung des Implantats, um beispielsweise einen günstigeren Implantationsort im Bereich der Schädeldecke zu wählen. Die große Anzahl an elektrischen Kontakten müssen aus dem Innenraum des hermetisch verschlossenen Implantatgehäuses herausgeführt werden, was bei einer großen Anzahl von Kontakten zu größeren Schwierigkeiten führen kann und die Größe und Bauform des Implantats massiv einschränkt. Je größer das Implantat ist, desto höher ist die Gefahr einer Entzündungsreaktion oder einer Abstoßung des Implantats beim Patienten.

Herkömmliche medizinische Implantate sind aus den Dokumenten EP 2 392 382 A1 und EP 2 123 325 A2 bekannt.

Es ist die Aufgabe der Erfindung, ein medizinisches Implantat mit einem Gehäuse, einer innerhalb des Gehäuses untergebrachten Steuerung sowie einer außerhalb des Gehäuses befindlichen Stimulationselektrode zur Verfügung zu stellen, welches gegenüber den nach dem Stand der Technik verfügbaren Implantaten verkleinert ist und das eine höhere Sicherheit für den Patienten ermöglicht, indem das Risiko für den Eintritt von Körperflüssigkeiten in das Gehäuse verringert wird. Die Konstruktion von Implantaten soll für die Ingenieure erleichtert werden. Das Implantat soll beim Patienten zu verringerten gesundheitlichen Schäden, wie beispielsweise Entzündungsreaktionen, oder einer verringerten Gefahr der Abstoßung führen.

Ausgehend vom Oberbergriff des Anspruchs 1 wird die Aufgabe erfindungsgemäß gelöst mit den im Kennzeichnenden Teil des Anspruchs 1 angegebenen Merkmalen.

Mit dem erfindungsgemäßen Gehäuse ist es nunmehr möglich, das Implantat gegenüber dem Stand der Technik zu verkleinern und die Betriebssicherheit für den Patienten zu erhöhen. Insbesondere kann das Risiko für einen Eintritt von Körperflüssigkeiten in das Implantat verringert werden. Eine Gesundheitsgefährdung für den Patienten, beispielsweise durch Entzündungsreaktionen oder durch die Abstoßung des Implantats, kann verringert werden. Für die Ingenieure, die Implantate entwickeln, wird die Konstruktion erleichtert.

Vorteilhafte Weiterbildungen der Erfindung sind in den Unteransprüchen angegeben.

Im Folgenden wird die Erfindung in Ihrer allgemeinen Form beschrieben.

Ein erfindungsgemäßes medizinisches Implantat umfasst ein Gehäuse, eine innerhalb des Gehäuses untergebrachte Steuerung sowie mindestens eine außerhalb des Gehäuses befindliche Stimulationselektrode. Das Gehäuse besteht mindestens in einem Teilbereich aus einem Isolatormaterial als Dielektrikum und das Isolatormaterial besitzt an der Innenseite und der Außenseite des Gehäuses jeweils mindestens eine elektrisch leitende Kontaktfläche, welche als Kondensatorelektrode fungiert, und zusammen mit dem Isolatormaterial einen Kondensator ausbildet. Zwischen der mindestens einen elektrisch leitenden Kontaktfläche an der Innenseite des Gehäuses und der mindestens einen elektrisch leitenden Kontaktfläche an der Außenseite des Gehäuses besteht keine elektrisch leitende Verbindung, wobei die mindestens eine elektrisch leitende Kontaktfläche an der Innenseite des Gehäuses Lamellen oder Platten aufweist, die von der Innenseite des Gehäuses in das Isolatormaterial hineinragen. Die mindestens eine elektrisch leitende Kontaktfläche weist an der Außenseite des Gehäuses Lamellen oder Platten auf, die von der Außenseite des Gehäuses in das Isolatormaterial hineinragen. Die Lamellen oder Platten der mindestens einen elektrisch leitenden Kontaktfläche an der Innenseite des Gehäuses und die Lamellen oder Platten der mindestens einen elektrisch leitenden Kontaktfläche an der Außenseite des Gehäuses sind alternierend angeordnet und bilden den Kondensator. Die Steuerung ist mit der mindestens einen elektrisch leitenden Kontaktfläche an der Innenseite des Gehäuses verbunden, um diese mit Strom zu versorgen. Die mindestens eine Stimulationselektrode ist mit der mindestens einen elektrisch leitenden Kontaktfläche an der Außenseite des Gehäuses elektrisch leitend verbunden.

Dem erfindungsgemäßen Gehäuse kommen die Funktionen zu, einen elektrischen Kondensator zu bilden und ein Eintreten von Körperflüssigkeit in das Implantat zu verhindern.

Das Gehäuse ist vorzugsweise ein Gehäuse für medizinisch implantierbare Pulsgeneratoren.

Die innere und äußere elektrisch leitende Kontaktfläche dient als Kondensatorelektrode und kann mit einer elektrischen Leitung in Kontakt gebracht werden, die sie in der Innenseite des Gehäuses mit Ladung beaufschlagen und an der Außenseite zu einer Veränderung der Ladung führen, die in eine Stimulationselektrode abgeleitet werden kann, mit der das medizinische Zielareal, beispielsweise eine Hirnregion oder der Herzmuskel mit einem elektrischen Reiz beaufschlagt werden soll. In einer Ausführungsform der Erfindung müssen an den elektrisch leitenden Kontaktflächen, die sich an der Außenseite des Gehäuses befinden, keine weiteren Stimulationselektroden angebracht sein. Dies ist beispielsweise dann der Fall, wenn das Implantat so implantiert werden kann, dass die Kontaktflächen selber als Stimulationselektroden dienen.

Dienen die äußeren elektrisch leitenden Kontaktflächen nicht selbst als Stimulationselektroden, so sind sie mit einer geeigneten Isolationsschicht, beispielsweise aus Silikon, zu versehen.

Im Gehäuse befinden sich die für die Funktion des Implantats notwendigen Bestandteile, wie beispielsweise eine Batterie und eine Steuerung, mit einer Leitung zu der elektrisch leitenden Kontaktfläche an der Innenseite des Implantats.

Erfindungsgemäß wird ein Gehäuse für ein medizinisches Implantat zur Verfügung gestellt, welches in dem baulichen Abschnitt, der als Kondensator ausgebildet ist, eine bevorzugte Kapazität zwischen 1 nF bis 1000 nF hat. Insbesondere liegt die Kapazität zwischen 5 nF und 1000 nF, besonders bevorzugt zwischen 5 nF und 500 nF. Die Werte ergeben sich aus Grenzen, die dadurch bestimmt werden, dass in das biologische Gewebe eine Ladungsmenge eingetragen wird, die einen Wert nicht übersteigen soll, der das Gewebe schädigt. Die untere Grenze des Wertes der Kapazität wird durch die Ladungsmenge bestimmt, die ausreicht, um eine physiologische Wirkung zu erzielen, nämlich beispielsweise den Herzmuskel ausreichend zu stimulieren, wenn das Implantat als Herzschrittmacher dient oder um in das Gehirn elektrische Reizmuster zu applizieren, die eine physiologischer Wirkung haben, beispielsweise die Unterdrückung einer neuronalen Aktivität oder die Minderung einer krankhaften Aktivität.

Vom Schutzbereich sollen daher alle Kondensatoren umfasst sein, die zu Werten für Kapazitäten führen, die diesen Zweck erfüllen.

Die Ladungsdichte soll zur Vermeidung von Gewebsschäden einen bestimmten Grenzwert nicht überschreiten. Dieser wird nach dem Stand der Technik beispielsweise bei maximal 30 $\mu$C/cm$^2$ angegeben. Daraus können mit der Information zur Dimension der Stimulationselektrodenoberfläche für typische Beispiele nach dem Stand der Technik maximale Ladungen von 1,8 $\mu$C für eine Stimulationselektrodenoberfläche von 0,06 cm$^2$ und 0,12 $\mu$C für eine Stimulationselektrodenoberfläche von 0,004 cm$^2$ abgeleitet werden. Bei einer angenommenen maximalen Spannung von 10 Volt werden somit 180 nF bzw. 12 nF für die Kapazität berechnet. Ein guter Wert der Kapazität für kleine Stimulationselektrodenoberflächen ist 10 nF.

Eine kleine Stimulationselektrodenoberfläche erlaubt nur die Applikation einer geringen Ladung pro Puls, wohingegen bei einer größeren Stimulationselektrodenoberfläche eine größere Ladung pro Puls appliziert werden kann, ohne Gewebsschäden zu riskieren. In der Praxis werden bei Implantaten nach dem Stand der Technik größere Kapazitäten verwendet als durch die Sicherheitsabschätzung begründet, da größere Kapazitäten eine höhere Energieeffizienz bei den Anwendungen aufweisen. Diese größeren Kapazitäten können jedoch auch bei der erfindungsgemäßen Vorrichtung verwirklicht sein.

Die bauliche Ausgestaltung des erfindungsgemäßen medizinischen Implantats orientiert sich an den physikalischen Grundlagen, mit denen die Kapazitäten erreicht werden können, die erfindungsgemäß verwirklicht werden sollen.

Nach Formel (1) gilt:

$$C = \varepsilon_0\, \varepsilon_r\, A/d \qquad (1)$$

mit

C = Kapazität (F)
A = Oberfläche der Kontaktfläche ($m^2$)
$\varepsilon_0$ = Permittivität im Vakuum
$\varepsilon_r$ = relative Permittivität des Isolators
d = Abstand der inneren Kontaktfläche zur äußeren Kontaktfläche, die mit dem Isolator den Kondensator ausbilden (m)

Durch geeignete Wahl der entsprechenden Größen für die in der Formel (1) angegebenen Parameter können Kombinationen von Größe und Abstand der elektrisch leitenden Kontaktflächen und notwendiger Permittivität des Isolatormaterials ermittelt werden, mit denen das erfindungsgemäße Gehäuse verwirklicht werden kann.

Auf Grund der Beziehung nach Formel (1) ist der Fachmann dazu in der Lage, Abmessungen und Materialen zu finden, die für den Bau des Gehäuses sinnvoll sind.

Die Größe der elektrisch leitenden Kontaktflächen kann sich in der Größenordnung von wenigen $mm^2$ befinden. Beispielseise ist ein typischer Wert 9 $mm^2$. Der Abstand der elektrisch leitenden Kontaktflächen darf die Stabilität des Gehäuses nicht negativ beeinträchtigen und kann beispielsweise 100 $\mu$m betragen.

Für den Isolator aus dem das Gehäuse gefertigt ist, wird Vorzugs weise Material gewählt, welches eine Permittivität zwischen 5 und 20000 aufweist.

Das Material sollte biokompatibel sein, also insbesondere für das menschliche Gewebe verträglich und nicht toxisch sein.

Dafür können Keramiken eingesetzt werden.

Beispielshaft aber nicht beschränkend können die Gehäuse aus Aluminiumoxidkeramik oder einer Keramik aus Yttriumstabilisiertem Zirkonoxid ($Zr_2O_3Y_2O_3$) bestehen.

Es können die bereits nach dem Stand der Technik bekannten Keramiken verwendet werden, die im Implantatbereich eingesetzt werden.

Es kommen auch geeignete monokristalline Kristalle in Betracht.

Es können auch Materialien eingesetzt werden, die künftig entwickelt werden und die geeignete Eigenschaften haben.

Als elektrisch leitende Kontaktstellen können metallische Elemente oder elektrisch leitende Stoffe eingesetzt werden. So können beispielsweise Platin-Iridium Elektroden verwendet werden.

Es kann jedes Material eingesetzt werden, das in der Medizin als Elektrodenmaterial bekannt ist und mit dem menschlichen Körper in Kontakt kommt. Es können auch Materialien eingesetzt werden, die künftig entwickelt werden und die geeignete Eigenschaften haben.

Die elektrisch leitenden Kontaktstellen bzw. Kontaktflächen können unterschiedlich ausgestaltet sein.

In der einfachsten Ausführungsform des erfindungsgemäßen Gehäuses für ein medizinisches Implantat befindet sich an der Innenseite des Gehäuses eine elektrisch leitende Kontaktstelle und an der Außenseite des Gehäuses eine weitere elektrisch leitende Kontaktstelle, die vorzugsweise planar sind und so zueinander angeordnet sind, dass sie in Verbindung mit dem Isolatormaterial des Gehäuses einen Kondensator mit der oben genannten Kapazität ausbilden.

Eine streng planare Ausführung der der elektrisch leitenden Kontaktflächen ist nicht zwingend. Es ist auch möglich, dass die elektrisch leitenden Kontaktflächen gewölbt sind.

Dann können die beiden elektrisch leitenden Kontaktflächen jeweils mit einer Leitung versehen werden, die elektrische Signale leiten kann.

Je nach Anwendung können auch mehrere elektrisch leitende Kontaktflächen an der Innenseite und an der Außenseite angebracht sein, die mit dem Material des Gehäuses einen Kondensator ausbilden. So können jeweils mindestens 2 elektrisch leitende Kontaktstellen an der Innenseite und der gegenüberliegenden Außenseite des erfindungsgemäßen Gehäuses angebracht sein. Die elektrisch leitenden Kontaktflächen sollten sich gegenüberliegen. Je mehr sie gegeneinander verschoben sind, je größer die Abweichung in der gegenüberliegenden Anordnung ist, desto geringer wird die Kapazität. Die Anzahl der elektrisch leitenden Kontaktstellen, die sich an der Innenseite und an der Außenseite befinden, ist frei wählbar und durch die Anwendung bestimmt, die mit dem Implantat durchgeführt werden soll. Beispielsweise können sich an der Innenseite und an der Außenseite des Gehäuses jeweils 1,2,3,4,8,40 oder 80 elektrisch leitenden Kontaktstellen befinden, die jeweils mit einer elektrischen Leitung in Verbindung stehen.

Der Abstand der elektrisch leitenden Kontaktflächen auf einer Seite untereinander sollte so bemessen sein, dass keine Ladung von einer elektrisch leitenden Kontaktfläche auf eine andere elektrisch leitenden Kontaktfläche überspringt.

So können in einem Beispiel vier in dem Gehäuse angebrachte elektrisch leitende Kontaktflächen mit vier Leitungen mit Strom versorgt werden und auf der den elektrisch leitenden Kontaktstellen gegenüberliegenden Seite des Gehäuses befinden sich vier weitere Kontaktflächen, an denen wiederum vier Leitungen angebracht werden, die den Strom an den Zielpunkt einer Stimulationselektrode leiten.

Die elektrisch leitenden Kontaktflächen sollen so dimensioniert sein, dass ihre Abmessungen im $\mu$m oder mm Bereich liegen, je nachdem wie viele Kontaktstellen vorhanden sein sollen.

Die elektrisch leitenden Kontaktstellen können auf die Oberfläche des Gehäuses aufgebracht sein, sie können dabei jedoch auch in das Gehäuse hineinragen.

In einer vorteilhaften Ausführungsform der Erfindung sind die elektrisch leitenden Kontaktflächen optimiert. Hierzu sind die Flächen, die als Kondensatorflächen wirksam werden, vergrößert. Dazu können die elektrisch leitenden Kontaktflächen in verschiedenen Geometrien zueinander angeordnet sein. Dem Fachmann sind derartige Anordnungen und die Methoden, mit denen sie ermittelt werden können, bekannt. Beispielsweise können die elektrisch leitenden Kontaktflächen lamellenförmig oder schichtartig angeordnet sein. Bei der schichtartigen Anordnung können Teile der elektrisch leitenden Kontaktstellen wie Zapfen in das Gehäusematerial hineinragen.

Das Gehäuse kann so ausgebildet sein, dass es überall die gleiche Dicke aufweist. Alternativ kann die Stelle des Gehäuses, an der sich die elektrisch leitenden Kontaktstellen befinden entsprechend der Funktion als Kondensator dünner oder dicker ausgebildet sein, als an anderen Stellen des Gehäuses.

Die Verwendung des erfindungsgemäßen Gehäuses für ein medizinisches Implantat, insbesondere für Pulsgeneratoren, hat den Vorteil, dass es mechanisch besonders stabil ist, Platz einspart und besonders sicher ist, da es keine Stellen gibt, in denen Flüssigkeit in das Gehäuse eintreten kann. Die Implantate können so klein werden, dass sie sogar in den Schädel eingebaut werden können. Beispielhafte Abmessungen sind 8mm x 40mm x 30mm. Gesundheitsschädigende Einwirkungen, wie Entzündungsreaktionen oder eine Abstoßung von Implantaten können verringert oder verhindert werden.

Die Figuren zeigen beispielhafte Ausgestaltungen des erfindungsgemäßen Gehäuses.

Es zeigt:

Fig.1: Eine Ausführungsform mit einer planaren Anordnung von elektrisch leitenden Kontaktflächen.

Fig.2: Eine Ausführungsform, bei der die elektrisch leitenden Kontaktflächen lamellenförmig in das Gehäuse hineinragen.

Fig.3: Eine Ausführungsform, bei der die elektrisch leitenden Kontaktflächen schichtartig in das Gehäuse hineinragen.

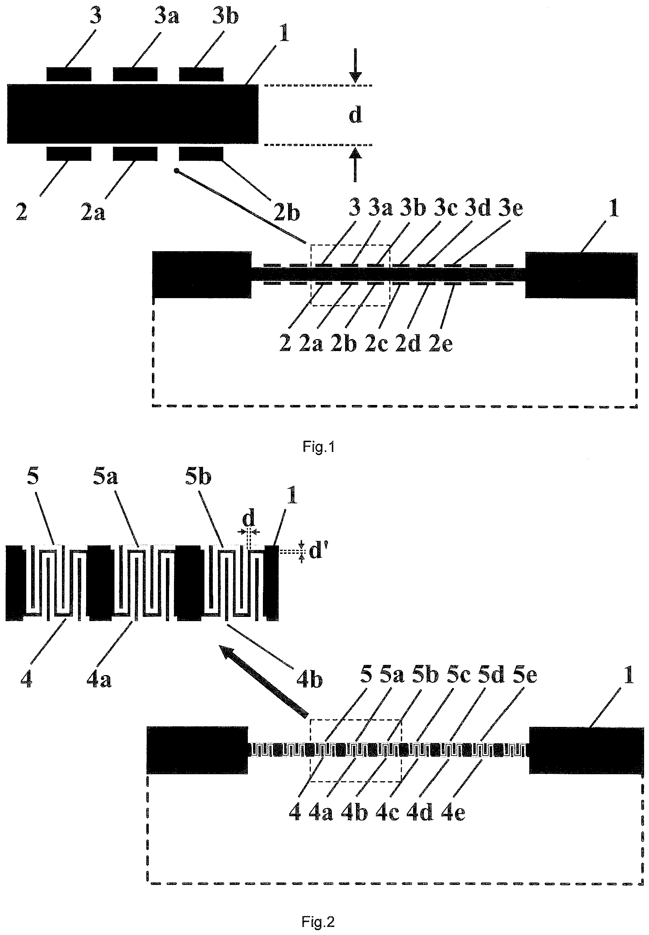

Figur 1 zeigt ein Gehäuse 1 für ein medizinisches Implantat, das in einem Bereich an der Innenseite elektrisch leitende Kontaktflächen 2, 2a,2b, 2c, 2d, 2e... und an der Außenseite elektrisch leitende Kontaktflächen 3, 3a, 3b, 3c, 3d, 3e... besitzt, die sich gegenüberliegen. Dazwischen befindet sich das Gehäuse 1, das als Dielektrikum ausgebildet ist, das einen Abstand d zwischen den Kontaktflächen herstellt. An jede der elektrisch leitenden Kontaktflächen 2, 2a, 2b, 2c, 2d, 2e... und 3, 3a, 3b, 3c, 3d, 3e... können elektrische Leitungen angebracht werden, die in der Figur jedoch nicht dargestellt sind. Die Leitungen, die außen angebracht sind, versorgen Endpunkte von Stimulationselektroden mit Strom. Die Leitungen, die innen angebracht sind, werden durch eine Steuerung mit Strom versorgt.

In Figur 2 wird das Gehäuse mit 1 bezeichnet. Die elektrisch leitenden Kontaktflächen 4, 4a, 4b, 4c, 4d, 4e... an der Außenseite und 5, 5a, 5b, 5c, 5d, 5e... an der Innenseite des Gehäuses 1 sind lamellenförmig ausgebildet und ragen in das Gehäuse 1 hinein. Dabei bilden die elektrisch leitenden Kontaktflächen in weißer Farbe dargestellte Schlangenlinien, die an der Oberfläche des Gehäuses 1 verlaufen und in das Gehäuse 1 hineinragen. Die elektrisch leitenden Kontaktflächen der Innenseite und der Außenseite des Gehäuses 1 greifen dabei ineinander wie Zahnräder. Dies kann dadurch erreicht werden, indem die elektrisch leitenden Kontaktflächen in das Gehäuse 1 bei dessen Produktion hineingebracht werden. Die Figur 2 stellt einen Querschnitt dar, so dass die lamellenartige Darstellung eine Projektion auf die Kante von planen, gebogenen elektrisch leitenden Kontaktflächen ist, die im Inneren des Gehäusematerials teilweise parallel zueinander angeordnet sind. Der Abstand d, d' zwischen den als plattenförmig ausgebildeten elektrisch leitenden Kontaktstellen ist in dem Vergrößerungsausschnitt der Figur 2 dargestellt. Die Abstände d und d' können gleich groß sein, sie können jedoch auch verschieden sein.

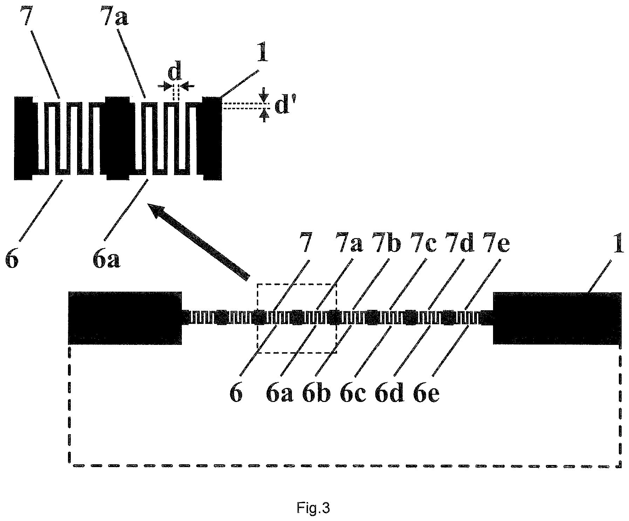

In Figur 3 sind die elektrisch leitenden Kontaktstellen 6, 6a, 6b, 6c, 6d, 6e... und 7, 7a, 7b, 7c,7d,7e.....als Platten ausgebildet, welche sich an der Innenseite und an der Außenseite des Gehäuses 1 befinden und die in das Gehäuse 1 in Form von Flächen hineinragen, die zu einer parallelen, schichtartigen Anordnung der elektrisch leitenden Kontaktstellen führen. Die elektrisch leitenden Kontaktstellen sind wiederum als Projektion auf den Schnitt des Gehäuses 1 dargestellt, so dass die sichtbaren weißen Linien die Projektion auf die Seite von bandförmigen elektrisch leitenden Kontaktstellen darstellt. Die elektrisch leitenden Kontaktstellen ragen alternierend in das Gehäuse 1 hinein und bilden parallel zueinander angeordnete Platten aus, deren Anstand d, d' die Kapazität des Kondensators bestimmt. Die Anstände d und d' können gleich oder verschieden sein.

Beispiel:

Berechnung der notwendigen Größe der Oberfläche, der metallischen Kontaktflächen und Stärke der Keramik.

Grundannahmen: Die relative Permittivität $\varepsilon_r$ der Keramik entspricht der von Bariumtitanat (BaTiO$_3$): bis zu 10000. Annahme 1: Pro Kontakt steht eine elektrisch leitende Kontaktfläche von 9 mm$^2$ (planar) bzw. 30 mm$^2$ (schichtweise) zur Verfügung. Daraus ergibt sich nach der Formel $C = \varepsilon_0\,\varepsilon_r\,A/d$ zur Erreichung einer Kapazität $C = 100$ nF eine Dicke der Keramikschicht von ca. 80 $\mu$m (bzw. 270 $\mu$m). Bei kleineren Elektroden-Kontaktflächen können auch kleinere Kapazitäten hinreichend sein, dadurch kann die minimale Dicke der Keramikschicht steigen, was die technische Realisierung erleichtert und die mechanische Stabilität erhöht.

## Patentansprüche

1. Medizinisches Implantat, umfassend:

ein Gehäuse (1), wobei das Gehäuse (1) mindestens in einem Teilbereich aus einem Isolatormaterial als Dielektrikum besteht und das Isolatormaterial an der Innenseite und der Außenseite des Gehäuses (1) jeweils mindestens eine elektrisch leitende Kontaktfläche (4, 4a, 4b...5, 5a, 5b...6, 6a, 6b...7, 7a, 7b...) besitzt, welche als Kondensatorelektrode fungiert, und zusammen mit dem Isolatormaterial einen Kondensator ausbildet, wobei zwischen der mindestens einen elektrisch leitenden Kontaktfläche (4, 4a, 4b...6, 6a, 6b...) an der Innenseite des Gehäuses (1) und der mindestens einen elektrisch leitenden Kontaktfläche (5, 5a, 5b...7, 7a, 7b...) an der Außenseite des Gehäuses (1) keine elektrisch leitende Verbindung besteht, wobei die mindestens eine elektrisch leitende Kontaktfläche (4, 4a, 4b...6, 6a, 6b...) an der Innenseite des Gehäuses (1) Lamellen oder Platten aufweist, die von der Innenseite des Gehäuses (1) in das Isolatormaterial hineinragen, wobei die mindestens eine elektrisch leitende Kontaktfläche (5, 5a, 5b...7, 7a, 7b...) an der Außenseite des Gehäuses (1) Lamellen oder Platten aufweist, die von der Außenseite des Gehäuses (1) in das Isolatormaterial hineinragen, und wobei die Lamellen oder Platten der mindestens einen elektrisch leitenden Kontaktfläche (4, 4a, 4b...6, 6a, 6b...) an der Innenseite des Gehäuses (1) und die Lamellen oder Platten der mindestens einen elektrisch leitenden Kontaktfläche (5, 5a, 5b...7, 7a, 7b...) an der Außenseite des Gehäuses (1) alternierend angeordnet sind und den Kondensator bilden; eine innerhalb des Gehäuses (1) untergebrachte Steuerung, die mit der mindestens einen elektrisch leitenden Kontaktfläche (4, 4a, 4b...6, 6a, 6b...) an der Innenseite des Gehäuses (1) verbunden ist, um diese mit Strom zu versorgen; und mindestens eine außerhalb des Gehäuses (1) befindliche Stimulationselektrode, die mit der mindestens einen elektrisch leitenden Kontaktfläche (5, 5a, 5b...7, 7a, 7b...) an der Außenseite des Gehäuses (1) elektrisch leitend verbunden ist.

2. Medizinisches Implantat nach Anspruch 1, **dadurch gekennzeichnet,** **dass** der Kondensator eine Kapazität zwischen 5 nF und 1000 nF besitzt.

3. Medizinisches Implantat nach Anspruch 1 oder 2, **dadurch gekennzeichnet,** **dass** das Isolatormaterial eine Keramik ist.

4. Medizinisches Implantat nach einem der Ansprüche 1 bis 4, **dadurch gekennzeichnet,** **dass** die elektrisch leitenden Kontaktflächen (4, 4a, 4b...5, 5a, 5b...6, 6a, 6b...7, 7a, 7b...) aus einer Platin-Iridium Legierung bestehen.

5. Medizinisches Implantat nach einem der Ansprüche 1 bis 5, **dadurch gekennzeichnet,** **dass** das Gehäuse (1) auf der Innenseite und auf der Außenseite jeweils mindestens 2 bis 80 Kontaktflächen (4, 4a, 4b...5, 5a, 5b...6, 6a, 6b...7, 7a, 7b...) besitzt.

## Claims

1. A medical implant comprising:

a housing (1), wherein the housing (1) is composed of an insulator material as a dielectric at least in a part region and the insulator material respectively has at least one electrically conductive contact surface (4, 4a, 4b...5, 5a, 5b...6, 6a, 6b...7, 7a, 7b...) at the inner side and outer sider of the housing (1), said electrically conductive contact surface acting as a capacitor electrode and forming a capacitor together with the insulator material; wherein there is no electrically conductive connection between the at least one electrically conductive contact surface (4, 4a, 4b...6, 6a, 6b...) at the inner side of the housing (1) and the at least one electrically conductive contact surface (5, 5a, 5b...7, 7a, 7b...) at the outer side of the housing (1); wherein the at least one electrically conductive contact surface (4, 4a, 4b...6, 6a, 6b...) at the inner side of the housing (1) has lamellae or plates which project into the insulator material from the inner side of the housing (1); wherein the at least one electrically conductive contact surface (5, 5a, 5b...7, 7a, 7b...) at the outer side of the housing (1) has lamellae or plates which project into the insulator material from the outer side of the housing (1); and wherein the lamellae or plates of the at least one electrically conductive contact surface (4, 4a, 4b...6, 6a, 6b...) at the inner side of the housing (1) and the lamellae or plates of the at least one electrically conductive contact surface (5, 5a, 5b...7, 7a, 7b...) at the outer side of the housing (1) are arranged alternately and form the capacitor;

a control which is accommodated within the housing (1) and which is connected to the at least one electrically conductive contact surface (4, 4a, 4b...6, 6a, 6b...) at the inner side of the housing (1) in order to supply power to said housing; and

at least one stimulation electrode which is located outside the housing (1) and which is electrically conductively connected to the at least one electrically conductive contact surface (5, 5a, 5b...7, 7a, 7b...) at the outer side of the housing (1).

2. A medical implant in accordance with claim 1, **characterized in that**
the capacitor has a capacity of between 5 nF and 1000 nF.

3. A medical implant in accordance with claim 1 or claim 2, **characterized in that**
the insulator material is a ceramic material.

4. A medical implant in accordance with any one of the claims 1 to 3, **characterized in that**
the electrically conductive contact surfaces (4, 4a, 4b...5, 5a, 5b...6, 6a, 6b...7, 7a, 7b...) comprise a platinum-iridium alloy.

5. A medical implant in accordance with any one of the claims 1 to 4, **characterized in that**
the housing (1) has at least 2 to 80 contact surfaces (4, 4a, 4b...5, 5a, 5b...6, 6a, 6b...7, 7a, 7b...) at the inner side and at the outer side.

## Revendications

1. Implant médical, comportant :

un boîtier (1), le boîtier (1) étant constitué au moins dans une zone partielle d'un matériau isolant à titre de diélectrique, et le matériau isolant comprenant sur le côté intérieur et sur le côté extérieur du boîtier (1) respectivement au moins une surface de contact électriquement conductrice (4, 4a, 4b ... 5, 5a, 5b ... 6, 6a, 6b .... 7, 7a, 7b ...) qui fait office d'électrode de condensateur, et qui réalise conjointement avec le matériau isolant un condensateur,

dans lequel

entre ladite au moins une surface de contact électriquement conductrice (4, 4a, 4b ... 6, 6a, 6b ....) sur le côté intérieur du boîtier (1) et ladite au moins une surface de contact électriquement conductrice (5, 5a, 5b ... 7, 7a, 7b ....) sur le côté extérieur du boîtier (1), il n'existe aucune liaison électriquement conductrice,

ladite au moins une surface de contact électriquement conductrice (4, 4a, 4b ... 6, 6a, 6b ....) sur le côté intérieur du boîtier (1) présente des lamelles ou des plaques qui pénètrent depuis le côté intérieur du boîtier (1) jusque dans le matériau isolant,

ladite au moins une surface de contact électriquement conductrice (5, 5a, 5b ... 7, 7a, 7b ....) sur le côté extérieur du boîtier (1) présente des lamelles ou des plaques qui pénètrent depuis le côté extérieur du boîtier (1) jusque dans le matériau isolant,

les lamelles ou les plaques de ladite au moins une surface de contact électriquement conductrice (4, 4a, 4b ... 6, 6a, 6b ....) sur le côté intérieur du boîtier (1) et les lamelles ou les plaques de ladite au moins une surface de contact électriquement conductrice (5, 5a, 5b ... 7, 7a, 7b ....) sur le côté extérieur du boîtier (1) sont agencées en alternance et constituent le condensateur ;

une commande logée à l'intérieur du boîtier (1), qui est connectée à ladite au moins une surface de contact électriquement conductrice (4, 4a, 4b ... 6, 6a, 6b ....) sur le côté intérieur du boîtier (1), pour l'alimenter en courant électrique ; et

au moins une électrode de stimulation située à l'extérieur du boîtier (1), qui est connectée de façon électriquement conductrice à ladite au moins une surface de contact électriquement conductrice (5, 5a, 5b ... 7, 7a, 7b ....) sur le côté extérieur du boîtier (1).

2. Implant médical selon la revendication 1, **caractérisé en ce que**
le condensateur présente une capacité comprise entre 5 nF et 1000 nF.

3. Implant médical selon la revendication 1 ou 2, **caractérisé en ce que**
le matériau isolant est une céramique.

4. Implant médical selon l'une des revendications 1 à 3, **caractérisé en ce que** les surfaces de contact électriquement conductrices (4, 4a, 4b ... 5, 5a, 5b ... 6, 6a, 6b .... 7, 7a, 7b ...) sont constituées d'un alliage de platine-iridium.

5. Implant médical selon l'une des revendications 1 à 4, **caractérisé en ce que** le boîtier (1) possède sur le côté intérieur et sur le côté extérieur respectivement au moins 2 à 80 surfaces de contact (4, 4a, 4b ... 5, 5a, 5b ... 6, 6a, 6b .... 7, 7a, 7b ...).

Fig.1

Fig.2

Fig.3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2392382 A1

- EP 2123325 A2

**In der Beschreibung aufgeführte Nicht-Patentliteratur**

- **ROBERT J. COFFEY.** Deep Brain Stimulation Devices: A Brief Technical History and Review. *Artificial Organs,* 2008, vol. 33 (3), 208-220

- **H.C.F. MARTENS.** Spatial steering of deep brain stimulation volumes using a novel lead design. *Clinical Neurophysiology,* 2011, vol. 122, 558-566